# EUROPEAN PATENT APPLICATION

(11) **EP 4 765 771 A1**
(43) Date of publication of application: **24.06.2026**
(21) Application number: 24222370.9
(22) Date of filing: 20.12.2024
(51) Int. Cl.: H04L 67/10, A61C 9/00, G16H 30/20

(54) **A METHOD FOR DENTAL SCANNING AND DENTAL SCANNING SYSTEM USING CLOUD COMPUTING**

(71) Applicant: 3Shape A/S, 1060 Copenhagen K (DK)
(72) Inventor: JENSEN, Lasse Skovgaard, 1060 Copenhagen K (DK); NAHSTOLL, Stefan, 1060 Copenhagen K (DK); JUUL, Frederik, 1060 Copenhagen K (DK); SCHOBER, Daniel, 1060 Copenhagen K (DK); WEST, Jelmer Te, 1060 Copenhagen K (DK)
(74) Representative: Zacco Denmark A/S

(57) **Abstract**

Disclosed is a computer-implemented method for facilitating 3D intraoral scanning using cloud computing. The method comprises obtaining scan data of a dental object at a local device, wherein the scan data comprises primary scan data indicative of the 3D geometry of the dental object of a patient, wherein the scan data comprises secondary scan data indicative of one or more characteristics other than the 3D geometry of the dental object. The method comprises establishing a connection between the local device and a remote computing device. The method comprises obtaining a performance indicator by detecting a performance of the connection. The method comprises evaluating the performance indicator and based on the evaluation. The method comprises, if the performance indicator exceeds a primary performance threshold, entering a full connection mode, wherein the full connection mode comprises transmitting the scan data from the local device to the remote computing device, receiving, at the local device from the remote computing device, a digital representation of the dental object computed from the transmitted scan data, and displaying the digital representation. The method comprises, if the performance indicator is less than the primary performance threshold entering a partial connection mode, wherein the partial connection mode comprises transmitting a reduced amount of the scan data from the local device to the remote computing device, receiving, at the local device from the remote computing device, a partial digital representation of the dental object computed from the transmitted reduced amount of scan data, and displaying the partial digital representation.

## Description

### Technical field

The disclosure relates to a computer-implemented method for facilitating 3D intraoral scanning using cloud computing, a computer program product for such a method, a non-transitory computer readable medium for such a method, and a scanning system for 3D intraoral scanning using cloud computing.

### Background

Intraoral scanning is a rapidly advancing technology in the field of dental and orthodontic care, enabling precise, digital capture of the oral cavity. Traditionally, dental impressions were taken using physical molds, where materials such as alginate or silicone were inserted into a patient's mouth to create negative impressions. These molds were then sent to a laboratory to be processed, a method that was not only time-consuming but also prone to errors due to material shrinkage, distortions, or improper handling.

The development of intraoral scanning systems offers a digital alternative that significantly enhances accuracy, speed, and patient comfort. By utilizing optical imaging technology, intraoral scanners capture 2D images of a patient's teeth and surrounding structures from which a digital 3D representation of the teeth can be created. This digital 3D representation can then be used for a variety of applications, such as designing crowns, bridges, dental implants, aligners, and for diagnostic purposes in orthodontics and prosthodontics.

Deriving the digital 3D representation from the captured 2D images may be a computationally heavy task requiring either a high-end laptop or desktop computer with a dedicated GPU. In addition to adding to the cost of the intraoral scanning system, this also imposes limitations on the mobility of the scanning system. Despite being a relatively small device, intraoral scanners are rarely used for work outside clinics as the computer needed for processing the scan data must be brought along. To mitigate this, the manufacturers of intraoral scanners are looking to cloud processing as a possible addition to the intraoral scanning system. By performing the heavy computations remotely, a scanning session can be performed from anywhere using only an intraoral scanner and a display device, e.g. a tablet, smart phone, or a standard laptop, provided the user has an internet connection with sufficient bandwidth to transfer the scan data.

One possible drawback of moving the heavier computations to the cloud, is that a scanning session may be interrupted if the user experiences a drop in the transfer rate, e.g. reduced bandwidth or latency issues, in their internet or Wi-Fi connection, which could lead to them not being able to complete a scan and having to send home a patient. This invention addresses the limitations of current intraoral scanning technologies by addressing how to manage unstable data connection during an intraoral scanning session with cloud computing.

### Summary

A first aspect of the present disclosure is to a computer-implemented method for facilitating three-dimensional (3D) intraoral scanning using cloud computing, the method comprising:
- obtaining scan data of a dental object at a local device, wherein the scan data comprises primary scan data indicative of the 3D geometry of the dental object of a patient, wherein the scan data comprises secondary scan data indicative of one or more characteristics other than the 3D geometry of the dental object;
- establishing a connection between the local device and a remote computing device;
- obtaining a performance indicator by detecting a performance of the connection;
- evaluating the performance indicator and based on the evaluation:
   if the performance indicator exceeds a primary performance threshold, entering a full connection mode, wherein the full connection mode comprises:
      - transmitting the scan data from the local device to the remote computing device,
      - receiving, at the local device from the remote computing device, a digital representation of the dental object computed from the transmitted scan data, and
      - displaying the digital representation,
   if the performance indicator is less than the primary performance threshold entering a partial connection mode, wherein the partial connection mode comprises:
      - transmitting a reduced amount of the scan data from the local device to the remote computing device,
      - receiving, at the local device from the remote computing device, a partial digital representation of the dental object computed from the transmitted reduced amount of scan data, and
      - displaying the partial digital representation.

Another aspect of the present disclosure is to a computer program product comprising instructions which, when the program is executed by a computer, cause the computer to carry out the method of the first aspect.

Another aspect of the present disclosure is to a non-transitory computer readable medium comprising instructions which, when executed by a computer, cause the computer to carry out the method of the first aspect.

Another aspect of the present disclosure is to a scanning system comprising:
- an intraoral scanner configured for obtaining scan data of a dental object, wherein the scan data comprises primary scan data indicative of the 3D geometry of the dental object of a patient, wherein the scan data comprises secondary scan data indicative of one or more characteristics other than the 3D geometry of the dental object;
- a display;
- a connection module configured for establishing a connection between the intraoral scanner and the display and a remote computing device, and for obtaining a performance indicator by detecting a performance of the connection; and
- one or more processors configured for:
   - evaluating the performance indicator and based on the evaluation:
      if the performance indicator exceeds a primary performance threshold, entering a full connection mode, wherein the full connection mode comprises:
         - transmitting the scan data to the remote computing device,
         - receiving a digital representation of the dental object computed from the transmitted scan data from the remote computing device, and
         - displaying the digital representation on the display,
      if the performance indicator is less than the primary performance threshold entering a partial connection mode, wherein the partial connection mode comprises:
         - transmitting a reduced amount of the scan data to the remote computing device,
         - receiving a partial digital representation of the dental object computed from the transmitted reduced amount of scan data from the remote computing device, and
         - displaying the partial digital representation on the display.

The scan data is obtained by capturing one or more two-dimensional (2D) images using an intraoral scanner. From the 2D images, 3D geometry may be calculated in a manner that depends on what type of scanning principle the intraoral scanner employs. The two principles that are most often used in intraoral scanners are focus scanning and triangulation scanning. In focus scanning, depth is determined based on capturing multiple images while linearly translating a focus lens and then identifying pixels of each of the multiple images that are in focus whereby the depth can be determined from the position of the lens and the associated focus distance at that position. In triangulation scanning, depth is determined by projections onto two or more planes, i.e. image and/or projector planes.

In addition to the geometry of the dental object indicated by the primary scan data, one or more other characteristics are also obtained from the 2D images. One of the most important other characteristics is texture, also referred to as color, which can be extracted by projecting probe light of multiple wavelengths, e.g. red, green, and blue, or white light, onto the dental object and capturing color data in the 2D images. The scan data may accordingly comprise image data divided into an array of pixels, where each pixel may have values related to position in space, such as x, y, and z values, i.e. the primary scan data, and values related to other characteristics, such as color.

The dental object may comprise multiple of the patient's teeth and even be a full arch of teeth or both arches. The field of view of the intraoral scanner is usually limited to a few square centimeters, so scanning the dental object fully requires moving the intraoral scanner around inside the patient's mouth while continuously acquiring sets of 2D images referred to as subscans from different positions and viewpoints. Scan data is obtained in each subscan, and the primary scan data of a subscan can be processed into 3D data, e.g. in the form of point cloud data, which can then be registered, also known as stitched, to the 3D data of previous subscans whereby a 3D representation of the dental object is gradually build up for each subscan acquired.

During a scanning session, a current digital representation, i.e. a representation made from subscans obtained up to that point of the parts of the dental object that has been scanned, will be displayed to show the user which parts of the patient's mouth have been scanned and which parts need to be scanned, i.e. the parts that are missing from the displayed digital representation. While a fully processed, final 3D representation of the dental object can be used for complicated dental procedures such as making dental prosthetics and therefore have high requirements on accuracy and color, the current digital representation only need to guide the user to complete the scan, i.e. collect subscans of the entire dental object, in real-time.

In a cloud computing assisted intraoral scanning session, scan data collected locally, i.e. at the local device, such as the intraoral scanner, is transmitted to the remote computing device which continuously processes the scan data as it is received to update the current digital representation with scan data as it comes in. The current digital representation is then passed back to the local device, where it can be displayed to guide the user through the scan. The current disclosure addresses how to handle situations where the user experiences connection issues during a cloud computing assisted intraoral scanning sessions, so that they do not need to abort the scan and ask the patient to come back again at a later time.

During a cloud computing assisted intraoral scanning session, the disclosed aspects continuously monitor the connection with the local device, e.g. an intraoral scanner and a local computer, and the remote computing device, i.e. the cloud. If a drop in connection quality is detected, e.g. an increase in latency or a drop bandwidth, the system or method will switch to the partial connection mode where the scanning session can be continued, albeit while transmitting a reduced amount of scan data from the local device to the remote computing device, resulting in a digital representation of the scanned parts of the dental object in a reduced quality, i.e. the partial digital representation, being displayed to guide the user through the scan. While displaying the partial digital representation may provide a less satisfactory user experience, it is preferable to not being able to complete the scan and having the patient come back another time. The disclosed aspects may comprise buffering the parts of the scan data that is not send while in the partial connection mode at the local device, and transmitting it after the scan, whereby the remote computing device may still generate a fully processed, final 3D representation with some delay.

For the purpose of this disclosure, the terms local and remote are defined as they are in computer science. A remote computing device, also known as a remote server, is a machine that is located elsewhere, i.e. not physically present for the user, and accessed over a network, usually the internet. Examples of remote computing devices are LAN servers and internet servers. A local device, also known as a client device, or simply client, is a machine that is physically present and directly accessible by the user. It can be a personal computer, laptop, or any device that the user is currently using.

### Brief description of the drawings

The above and other features and advantages of the present invention will become readily apparent to those skilled in the art by the following detailed description of exemplary embodiments thereof with reference to the attached drawings, in which:
FIG. 1a shows a perspective view of an intraoral scanner;
FIG. 1b shows a cross-sectional view of an intraoral scanner;
FIG. 2 shows a block diagram of a scanning system;
FIG. 3 shows a scanning system;
FIG. 4 shows an illustration of a scanning session at the local device;
FIG. 5 shows a graphical user interface;
FIG. 6 shows a final 3D representation;
FIG. 7 shows a flowchart of a computer implemented method of the invention;
FIG. 8 illustrates the contributors of latency in a cloud scanning system;
FIG. 9 shows a flowchart of a round-trip time measurement;
FIG. 10 shows a method for determining event-to-display latency; and
FIG. 11 shows a method comprising multiple increasingly invasive partial connection modes.

### Detailed description

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

While the dental object is referred to in singular, the dental object may be a group of dental features of a subject's, i.e. patient's, mouth, such as a group of teeth and their encircling gingiva. Examples of dental objects include any one or more of: tooth/teeth, gingiva, implant(s), dental restoration(s), dental prostheses, edentulous ridge(s), and/or combinations thereof. Alternatively, the dental object may be a gypsum model or a plastic model representing a subject's teeth. As an example, the dental object may comprise teeth and/or gingiva of a subject. The dental object may only be a part of the subject's teeth and/or oral cavity, since the entire set of teeth of the subject is not necessarily scanned during a scanning session. A scanning session may be understood herein as a period of time during which data (such as 2D images) of the dental object is acquired/obtained.

To obtain scan data, an intraoral scanner for acquiring images within an intraoral cavity of a patient is used. The intraoral scanner is a handheld intraoral scanner, i.e. a device configured to be held with a human hand. The intraoral scanner comprises one or more projector unit(s) configured for illuminating the dental object with probe light. The intraoral scanner comprises one or more camera unit(s) configured for acquiring 2D images of the dental object. The intraoral scanner may employ any suitable scanning principle such as triangulation-based scanning, stereo vision, structure from motion, confocal scanning, focus scanning, time-of-flight scanning, or other scanning principles. In some embodiments, the intraoral scanner employs a triangulation-based scanning principle. As an example, a projector unit and one or more camera units may be utilized to determine points in 3D space based on triangulation. In other embodiments, the intraoral scanner employs a focus-based scanning principle. A focus intraoral scanner is further described in EP 2 442 720 B1 by the same applicant.

A projector unit may be understood herein as a device configured for projecting light onto a surface, such as the surface of a dental object. In preferred embodiments, the projector unit is configured to project a pattern of light onto the surface of a dental object. The projector unit may be configured to project a pattern of light such that the pattern of light is in focus at a predefined focus distance measured along a projector optical axis. Features in the pattern of light may be used to extract information on the 3D geometry from the 2D images, e.g. by finding correspondence between a camera pixel in one camera unit observing a particular pattern feature and a projector pixel in the projector unit that generated that particular pattern feature unit or a camera pixel in another camera unit also observing that particular pattern feature, whereby triangulation may be performed.

Each projector unit may comprise one or more light sources. The light source(s) may be configured to generate light of a single wavelength (monochromatic), a distribution, e.g. a Poisson distribution, of wavelengths, or a combination of wavelengths (polychromatic). The combination of wavelengths may be produced by a light source configured to produce light comprising different wavelengths (such as white light). A light source of the one or more light sources may be configured to generate light at other wavelengths than at least some of the other light sources of the one or more light sources, e.g. the one or more light sources may comprise a light source generating red light, a light source generating blue light, and a light source generating green light. In some embodiments, each projector unit comprises a light source for generating white light. Alternatively, the projector unit may comprise multiple light sources such as LEDs individually producing light of different wavelengths (such as red, green, and blue) that may be combined to form light comprising different wavelengths. Thus, the light produced by the light source(s) may be defined by a wavelength defining a specific color, or a range of different wavelengths defining a combination of colors such as white light. In some embodiments, the light source is a diode, such as a white light diode, or a laser diode.

In some embodiments, the intraoral scanner comprises a light source configured for exciting fluorescent material to obtain fluorescence data from the dental object such as from teeth. Such a light source may be configured to produce a narrow range of wavelengths. In other embodiments, the intraoral scanner comprises an infrared light source, which is configured to generate wavelengths in the infrared range, such as between 700 nm and 1.5 µm. In some embodiments, the intraoral scanner comprises one or more light sources selected from the group of: Infrared (IR) light source, near-infrared (NIR) light source, blue light source, violet light source, ultraviolet (UV) light source, and/or combinations thereof. In some embodiments, the intraoral scanner comprises a first light source forming part of the projector unit, and one or more second light sources, e.g. I R-LED(s) and/or UV-LED(s), located in a distal part of the intraoral scanner, such as in the tip of the intraoral scanner.

A camera unit may be understood herein as a device for capturing a 2D image of the dental object. Each camera unit may comprise an image sensor for generating an image based on incoming light e.g. received from the illuminated dental object. The image sensor may comprise a 2 dimensional array of camera pixels. Each camera pixel may comprise one or more subpixels configured to capture light of a certain wavelength or wavelength interval, whereby a camera pixel may capture color data by adding the values of at least some of its associated subpixels. One or more of the subpixels may be configured for capturing light outside the visible spectrum, e.g. UV and/or IR wavelengths.

As an example, the image sensor may be an electronic image sensor such as a charge-coupled device (CCD) or an active-pixel sensor (CMOS sensor). Each image sensor may define an image plane, which may be understood as the plane that contains the object's projected image. Each image obtained by the image sensor(s) may comprise a plurality of image features, wherein each image feature originates from a pattern feature of the projected pattern. In some embodiments, one or more of the camera units comprise a light field camera. Preferably, each camera unit defines a camera optical axis. The camera units may further comprise one or more focus lenses for focusing light.

In some embodiments, the image sensor is a monochrome image sensor, wherein each pixel is associated with a single color channel, e.g. is a grayscale color channel, wherein the value of each pixel represents only an amount of light. In other embodiments, the image sensor is a color image sensor or an image sensor comprising a color filter array on the array of pixels. As an example, the color filter array may be a Bayer filter employing an arrangement of four color filters: Red (R), Green (G), Green (G), and Blue (B). The Bayer filter may also be referred to as an RGGB filter. When utilizing the image sensor data, color pixels may be combined to monochrome pixels of 2 x 2 color pixels for 3D depth reconstruction. In this case, the resolution of the 3D depth reconstruction is only half the resolution of the image sensor in each direction. When obtaining texture (color) images the full native resolution is preferably utilized (with color filtered pixels).

The image sensor may have an image frame rate of at least 30 frames per second, such as at least 60 frames per second, or even at least 90 frames per second. At least some of the frames may be dedicated to obtaining primary scan data. In frames dedicated to obtaining primary scan data, the one or more projector units may be configured for probe light comprising one or more discrete wavelengths of light, e.g. by light generated from one or more laser light sources. At least some of the frames may be dedicated to obtaining secondary scan data. At least some of the frames dedicated to obtaining secondary scan data may be dedicated to obtain fluorescence data. The one or more projector units may be configured for emitting UV light during frames dedicated to obtain fluorescence data. At least some of the frames dedicated to obtaining secondary scan data may be dedicated to obtain IR data. The one or more projector units may be configured for emitting IR light during frames dedicated to obtain fluorescence data. At least some of the frames dedicated to obtaining secondary scan data may be dedicated to obtain texture/color data. The one or more projector units may be configured for emitting white light during frames dedicated to obtain fluorescence data. Alternatively, the one or more projector units may be configured for emitting monochromatic light of a first wavelength, e.g. green, during some of the frames dedicated to obtain fluorescence data, and for emitting monochromatic light of a second wavelength, e.g. blue, during some of the frames dedicated to obtain fluorescence data, and for emitting monochromatic light of a third wavelength, e.g. red, during some of the frames dedicated to obtain fluorescence data, whereby the color(s) of the dental object may be calculated by adding the frames dedicated to obtain fluorescence data.

The scan data disclosed herein may be 2D images obtained by the intraoral scanner or data based on these 2D images. As described above, scan data is continuously obtained during the scanning session by performing multiple subscans taken at different positions and orientations of the intraoral scanner. Scan data from a subscan may comprise primary scan data, which may be used to calculate 3D geometry, and secondary scan data, which may be used to calculate other characteristics, for the dental object when observed from the position and orientation of that particular subscan. When scan data for a subscan has been acquired, it must be registered to the scan data of previous subscans whereby a 3D digital representation will gradually be built.

The primary scan data refers to the parts of the scan data which are used to derive the 3D geometry of the dental object. The primary scan data may be in the form of raw 2D images captured from the one or more camera units, in which case the 3D geometry must be calculated by the remote computing device, and subsequently also registered to the 3D geometry extracted from the accumulated, previous scan data. Alternatively, the local device may perform initial processing on the 2D images to generate primary scan data in the form of a depth map, i.e. an image that contains information relating to the distance of the surfaces of scene objects from a viewpoint, i.e. the intraoral scanner. In this case, the remote computing device needs to register the depth map to the depth maps from the accumulated, previous scan data.

The secondary scan data refers to the parts of the scan data which are used to derive characteristics other than the 3D geometry of the dental object. The most important of these is the color(s), also referred to as texture, of the dental object. As described above, color data may be collected by sequentially switching between light sources of different wavelength and capturing the wavelengths either by different image sensor sensitive to different colors, e.g. R, G, and B, or in different image frames, or by using white light, optionally also in image frames dedicated to capturing color data.

Other types of secondary data may be IR data and/or fluorescence data, collectively referred to as diagnostics data. IR data may be collected by using a light source generating IR light and an image sensor sensitive to IR wavelength, such as Near Infrared (NIR) and/or Short Wavelength Infrared (SWIR). IR data may be used to detect dental conditions such as caries, particularly proximal caries, i.e. caries between teeth, as IR may penetrate at least part of the enamel. The light source generating IR light may be configured for generating infrared light in the wavelength interval 700-1500nm. Fluorescence data may be collected by using a light source generating UV light and an image sensor sensitive to the wavelength of bacteria fluorescence, about 370-600nm. Fluorescence data, also referred to as UV data due to the wavelength used for excitation, may be used to detect dental conditions such as caries or plaque which are caused by the accumulation of bacteria. IR data and fluorescence data may be gathered by using image sensors dedicated for the relevant wavelengths, and/or by dedicating image frames for the collection of IR data and/or fluorescence data.

The primary and secondary data may be packaged separately, or it may be gathered in the same data structure, e.g. a matrix representing pixels of the image sensor(s) where each cell may have a list of values, such as x, y, and z in the coordinate system of the intraoral scanner, and texture and/or diagnostics data, such as R, G, and B, and IR or UV values. For the purpose of guiding the user through a cloud assisted scan by displaying a digital representation, the invention prioritizes transmitting the primary scan data, so that the remote computing device can process the 3D geometry, register it to previous scan data to update the digital representation, and return it to local device for displaying so that the user can see their progress. While texture data may increase the user experience, the inventors found that it is not essential for the purpose of guiding the user through the scan.

The connection established between the local device and the remote computing device at least requires that and operational connection through a Wide Area Network (WAN), e.g. the internet, is established. It is important to note that the local device may comprise multiple sub devices, e.g. an intraoral scanner and a local computing device with a display. The connection established between the local device and the remote computing device may therefore comprise multiple channels and/or networks. The connection may further comprise one or more connection(s) on a Local Area Network (LAN), e.g. through Wi-Fi or ethernet, whereby the sub devices may communicate directly via the LAN network. This configuration is however not a requirement, and the invention can also work by each or some of the sub devices having their own connection to the remote computing device, whereby communication between the sub devices goes through the remote computing device, an example of such a configuration could be the intraoral scanner and local computing device having independent cellular connection, e.g. 5G, connecting them to the internet and the remote computing device. However, the most common implementation of the invention will have the sub devices on the same LAN network, e.g. through Wi-Fi, with the LAN being connected to the internet and therethrough to the remote computing device.

Because the combined connection has these successive links, the connection quality will also be defined by the combined performance of the connection links. This makes the disclosed invention all the more advantageous as the combined connection between the local device and the remote computing device has multiple points of failure, and a drop in performance in any one of the connection links will result in a bottleneck affecting the combined connection.

Several metrics may influence the quality of the connection. Amongst the most important are the network bandwidth, which is the maximum rate of data transfer across the connection, and latency, e.g. the accumulated delay caused by the process steps between obtaining scan data and displaying the digital representation or partial digital representation. If the network bandwidth is an issue, the user will experience it as if it were a latency issue, simply because of the delay caused by transmitting more data than the connection can support.

Response time (also referred to as round-trip latency) is an important aspect of cloud scanning that directly affects the user experience. One way to describe response time is the amount of time that passes between the moment at which the user performs an action, e.g. pressing a button on an intraoral scanner, obtaining a subscan, and the moment at which a result of that action is displayed to the user (e.g. scan starts/stops, subscan is added). Response times may be affected by any of the various sources of latency discussed below. Most sources of latency fall into two categories: processing latency and network latency.

Processing latency is a result of the amount and quality of processing resources dedicated to a particular scanning session, and may be affected by the number, speed, and efficiency of processors or processing cores assigned to process user inputs and render corresponding responses. Processing latency may further be affected by the complexity of user inputs (e.g. only 2D data vs. high-accuracy mode). Processing latency may be negatively affected by load, i.e. the number of simultaneous users, as this means that the total amount of processing power is divided over more users. While processing latency is inherently a limitation dependent on the hardware of the remote computing device, it is considered as part of the performance of the connection for the sake of this disclosure.

Network latency is a result of the quality of the communication network(s) being used to support the cloud scanning session and may be affected by any number of external factors, such as interference, or internal factors, such as varying levels of traffic and available bandwidth. As the distance between a user and a particular scanning server system increases, the amount of physical network elements required to support the scanning session increases, which adds the potential for more network latency to be introduced. For example, a first user located many miles away from a data center housing a particular scanning server may experience more latency during an online session hosted by the scanning server than a second user taking part in the same session but located across the street from the data center.

In some wireless communications systems, such as fifth generation (5G) New Radio (NR) systems, a wireless communication network may support techniques for round-trip time (RTT) as a latency performance indicator. For example, the network, e.g. a 5G system (5GS), may support latency sensitive applications, such as intraoral scanning applications. In such applications, an intraoral scanner may transmit an uplink message, e.g. subscan or inertial measurement unit (IMU) data, to the network and, in response, may receive downlink messages, e.g. encoded video, corresponding to the transmitted uplink message. In some examples, an RTT pertaining to the uplink messages and the corresponding downlink messages may determine the user experience for such applications. As such, an application server, e.g. hosting the application, may specify an RTT latency requirement to the network, e.g. a base station, or a network core, and the network, e.g. or the application server, may determine one or multiple one-way directional delay budgets, e.g. an uplink delay budget, a downlink delay budget, or both, that satisfy the RTT latency requirement.

Packet loss may also be an issue, particularly if the connection comprises one or more wireless links in the connection chain, e.g. Wi-fi or 5G. The scan data may be transmitted as data packages comprising error detection and/or error correction data, whereby minor packet loss issues can be mitigated. If the packet loss rate is above an acceptable threshold, the invention may comprise sending multiple copies of the primary and/or secondary scan data.

In light of the above, the performance indicator may therefore be a measure of one or more of the above-mentioned network quality metrics. Because an issue with many of these metrics will come into expression as an increase in RTT latency, RTT latency may be the metric measured in the step of obtaining a performance indicator by detecting a performance of the connection. Measuring RTT latency may comprise measuring the time between the transmission of the scan data or the reduced amount of the scan data and the reception of the digital representation or partial digital representation resulting from that data, e.g. by adding time-stamp data at the local device before transmission.

Obtaining a performance indicator by detecting a performance of the connection may also comprise detecting the performance of LAN network, particularly wireless connections in the LAN network, which connects local device to the internet. If the performance indicator indicates that the performance of the LAN network is below a LAN threshold, the aspects of the invention may comprise outputting, e.g. by displaying, a warning indicator with a prompt for the user to change to a wired connection, i.e. request that the user changes from Wi-Fi to wired at the local part of the connection chain.

Evaluating the performance indicator and based on the evaluation may depend on the rate at which scan data is being obtained compared to the bandwidth of the connection. This will depend on the scanning principle, and also the number of camera unit(s), being used and the amount of local processing of the 2D images being performed at the local device. The primary performance threshold may therefore be based on the rate at which scan data is being obtained compared to the bandwidth of the connection. When the performance indicator is an indicator of connection bandwidth, the primary performance threshold may be a value between 30 and 70 mbit/s, such as between 40 and 60 mbit/s.

As previously mentioned, most network issues will come into expression as delay. Obtaining a performance indicator by detecting a performance of the connection may therefore comprise measuring a RTT latency, and evaluating the performance indicator may comprise comparing the measured RTT latency to a time threshold comprised by the primary performance threshold. If latency or RTT latency is used as the performance indicator, the time threshold comprised by the primary perform may be in the range 20-200ms, preferably 20-100ms, more preferably 20-60ms. The performance indicator may be an aggregate of multiple connection metrics, e.g. by scoring each metric to obtain a combined score, and evaluating the performance indicator may comprise comparing the aggregate to the primary performance threshold.

At its core, the purpose of intraoral scanning, be it ordinary or cloud assisted, is to generate a digital 3D representation of the dental object. When completed, the digital 3D representation can be inspected to aid the user, e.g. a dentist, in identifying dental disorders in the patient, planning dental treatments, or as a basis for designing dental prosthetics. Before completion, i.e. as the scan is ongoing, the digital representation or partial digital representation may be displayed as a 3D visualization and/or as a 2D visualization in a Graphical User Interface (GUI) so that the user can see which parts of the dental object has been scanned and which still needs to be scanned. The digital representation and/or partial digital representation may also comprise a viewfinder image depicting the current field of view of the intraoral scanner.

Generating the digital representation and/or partial digital representation may comprise updating an existing digital representation and/or an existing partial digital representation made from previous scan data, i.e. scan data acquired earlier in the scan session. To put it in other words, computing the digital representation of the dental object and computing the digital representation of the dental object may be based on earlier scan data from the ongoing scanning session.

The rate at which the digital representation or partial digital representation is generated and transmitted to the local device for displaying may depend on the rate at which subscans are acquired and/or the refresh rate of the display. If the refresh rate of the display is faster than the rate at which subscans are acquired, the digital representation or partial digital representation cannot be updated faster than the rate at which subscans are acquired. The digital representation or partial digital representation may therefore be generated/updated at the rate at which subscans are acquired, while the digital representation or partial digital representation may be transmitted and/or displayed at the refresh rate of the display, where some of the transmitted and/or displayed digital representation or partial digital representation will be repetitions of the former digital representation or partial digital representation because the rate at which subscans are acquired represents a bottleneck for refreshing the display.

If the refresh rate of the display is slower than the rate at which subscans are acquired. The digital representation or partial digital representation may therefore be generated/updated at the rate at which subscans are acquired, while the digital representation or partial digital representation may be transmitted and/or displayed at the refresh rate of the display, where some of the generated/updated digital representations or partial digital representations are not transmitted and/or displayed because the refresh rate of the display represents a bottleneck for refreshing the display.

In the full connection mode, the digital representation may have a first color depth, e.g. higher than 8bit, such as 24bit, wherein the colors are calculated based on the secondary scan data. In the full connection mode, the digital representation may have a first resolution. In the partial connection mode, the digital representation may have a second color depth, e.g. 8bit or less, such as 8bit or 4bit, wherein second color depth is lower than the first color depth. In the partial connection mode, the partial digital representation may have a second resolution, wherein second resolution is lower than the first resolution. Additionally or alternatively the amount of color data may be reduced by only including color data in the data transmitted to the remote computing device in part of the subscans/frames, e.g. transmitting the scan data obtained in every second subscan including color data and every other second in monochrome.

Disclosed is a computer-implemented method for facilitating 3D intraoral scanning using cloud computing, the method comprising:
- obtaining scan data of a dental object at a local device, wherein the scan data comprises primary scan data indicative of the 3D geometry of the dental object of a patient, wherein the scan data comprises secondary scan data indicative of one or more characteristics other than the 3D geometry of the dental object;
- establishing a connection between the local device and a remote computing device;
- obtaining a performance indicator by detecting a performance of the connection;
- evaluating the performance indicator and based on the evaluation:
   if the performance indicator exceeds a primary performance threshold, entering a full connection mode, wherein the full connection mode comprises:
      - transmitting the scan data from the local device to the remote computing device,
      - receiving, at the local device from the remote computing device, a digital representation of the dental object computed from the transmitted scan data, and
      - displaying the digital representation,
   if the performance indicator is less than the primary performance threshold entering a partial connection mode, wherein the partial connection mode comprises:
      - transmitting a reduced amount of the scan data from the local device to the remote computing device,
      - receiving, at the local device from the remote computing device, a partial digital representation of the dental object computed from the transmitted scan data, and
      - displaying the partial digital representation.

The advantage of providing a full connection mode and a partial connection mode, and the possibility of adaptively switching between them, is that the user is provided with the advantages of cloud computing, such as lower requirements of their own, local hardware, which also allows for increased mobility as the local hardware may be moved around more easily, while also mitigating the risk of running into connection issues, which in the worst case may lead to the user having to postpone treatments meaning that both the user, i.e. the dental practitioner, and the patient will waste time.

The full connection mode comprises, by the remote computing device, computing the digital representation of the dental object based on the scan date. The partial connection mode comprises, by the remote computing device, computing the digital representation of the dental object based on the reduced amount of the scan data.

The partial connection mode may comprise:
- generating the reduced amount of scan data by:
   ∘ compressing at least a part of the scan data,
   ∘ reducing a resolution of the primary scan data, and/or
   ∘ omitting at least part of the secondary scan data from the scan data.

The steps taken to reduce the amount of scan data may depend on the severity of the connection issue. For smaller connection issues compressing at least a part of the scan data, e.g. using dynamic compression with a compression ratio selected based on the performance indicator, may suffice and, depending on the selected compression ratio, result in a partial digital representation almost indistinguishable in quality from the digital representation produced in the full connection mode.

Compressing at least a part of the scan data may be performed at a compression ratio determined based on the performance indicator. Certain compression algorithms, such as H265, utilize a compression parameter, a so-called Q-factor, that balances the file size and overall quality of the file after decompression. The compression parameter can be linked to the internet and/or Wi-Fi connection speed, e.g. by selecting the compression parameter based on the performance indicator, to maintain a constant reliable stream of information without having to resort to omitting scan data from the transmitted data. The dynamic compression factor, e.g. the Q-factor, can be used as an input argument for the reconstruction algorithm of the digital representation. The input can be used as a basis for a weighted average for the reconstruction of the textures on the model. As such this embodiment is not just cloud-scanning specific but is only of value for that application. Optimal color support is of course ideal, and simple to realize with a local machine for running the reconstruction.

It must be noted that the full connection mode may also perform data compression, although it will be using compression with little or no loss in quality. The main reason for this is that compressed 2D images as opposed to uncompressed images results in higher quality textures because one can send a larger number of pixels, over which the color can be averaged, as opposed to sending only a few images which the texture must be based on. A balance needs to be achieved however, to not over-compress and lose important details, but in general compression is a useful tool for all connections, except the rare cases where connection bandwidth far exceeds the amount of scan data produced. Thus, in an embodiment, both the scan data and the reduced amount of scan data is compressed, wherein the scan data is compressed at a lower compression ratio than the reduced amount of scan data.

The secondary scan data may comprise color data indicative of one or more colors of the dental object. Omitting at least part of the secondary scan data from the scan data may comprise reducing the color depth of the color data or omitting the color data. In the partial connection mode, color data may be sent in a different color space and/or a reduced bit rate. The partial 3D representation may be in a reduced color depth or monochrome. Bigger connection issues will inevitably be noticeable by the user and require that bigger measures are taken in the partial connection mode. Such measures may be the omission of at least part or all of the secondary scan data from the scan data, such as color data, which may lead to a partial digital representation of reduced color depth, e.g. greyscale or black and white, or with artificially generated colors. While this will reduce the user experience it is still much preferred to the alternative of canceling the scanning session.

The secondary scan data may comprise infrared (IR) data. Omitting at least part of the secondary scan data from the scan data comprises omitting the IR data. The secondary scan data may comprise fluorescence data. Omitting at least part of the secondary scan data from the scan data may comprise omitting the fluorescence data. While the diagnostics data is highly useful when the user inspects the final 3D representation for diagnostics purposes, it provides little when guiding the user through the scan. It is therefore advantageous to omit parts or all of the diagnostics data from the reduced amount of the scan data to save the limited connection bandwidth for the primary scan data, and optionally the color data.

The secondary scan data may comprise voice data comprising voice recordings of a user recorded during the step of obtaining scan data of the dental object. Omitting at least part of the secondary scan data from the scan data may comprise omitting the voice data. Voice data may be recorded concurrently with capturing the 2D images, whereby the user may leave verbal comments of what they observe in the patient's mouth while scanning for journaling purposes. Similar to the diagnostics data, voice data may be useful, particularly when inspecting the final 3D representation after some time has passed from the scan. It does however not help in guiding the user through the scan, and it is therefore advantageous to omit voice data from the scan data when generating the reduced amount of the scan data.

Detecting the performance of the connection may comprise:
- transmitting time stamp data from the local device to the remote computing device, and
- receiving a result of transmission time measurement computed from the transmitted time stamp data from the remote computing device.

This is advantageous as it allows the remote computing device to calculate the uplink, i.e. from the local device to the remote computing device, speed and identify if there are any connection issues in the uplink. Similarly, detecting the performance of the connection may comprise, by the local device, receiving time stamp data from the remote computing device, and performing, at the local device, a transmission time measurement based on the received time stamp data from the remote computing device, whereby a downlink, i.e. from the remote computing device to the local device, speed may be derived. Isolating the connection issue to one of the uplink or downlink may be useful when selecting what measures to take in dealing with the connection issue. If the connection issue is an uplink issue, the method/system may reduce the amount of scan data in the manners disclosed above, whereas if the connection issue is a downlink issue, the method/system may maintain transmission of the scan data from the local device as if it was operating in the full connection mode, while reducing the quality of the partial digital representation to reduce the load on the downlink.

Detecting the performance of the connection may comprise:
- transmitting a return data package from the local device to the remote computing device,
- at the local device, receiving the return data package from the remote computing device, and
- calculating a round-trip time based on the time difference between when the return data package was transmitted and received at the local device.

Because any issue in the connection between the local device and the remote computing device will affect the RTT latency it is the best metric to measure the connection's overall performance. While measuring the RTT latency may not help in isolating the cause of a connection, it is an efficient way to determine if there are any bottlenecks in the connection, be it uplink, downlink, or processing at the remote computing device. From a user perspective, it does not matter where the issue is, the only thing the user experiences is a delay in the RTT latency. It is therefore also the best metric to base the performance indicator on for the determination on whether to operate in the full connection mode or the partial connection mode.

Detecting the performance of the connection may comprise measuring a data packet loss rate between the local device and the remote computing device. Detecting the performance of the connection may comprise measuring a data packet error rate between the local device and the remote computing device. Measuring the data packet loss rate in the uplink or downlink is a useful alternative or addition to measuring latency as it may reveal issues not detectable in latency measurements. Latency measurements mainly detect the speed/rate of which process steps takes, it does not necessarily detect if data is being lost during these process steps. Packet loss may be detected by adding error detection data, such as a checksum, so that scan data can be validated when received by the remote computing device, and/or similarly adding error detection data to the digital representation or partial digital representation when sending it from the remote computing device to the local device.

Generating the reduced amount of scan data may comprise:
∘ if the performance indicator is between the primary performance threshold and a secondary performance threshold which is lower than the primary performance threshold, compressing at least a part of the scan data, preferably all of the scan data, at a dynamic compression ratio selected based on the performance indicator, and
∘ if the performance indicator is less than the secondary performance threshold, omitting at least a part of the secondary scan data from the scan.

Introducing a multistep approach allows the system/method to take the measures needed depending on the severity of a connection issue, without resorting to measures that are more drastic than required. It is therefore advantageous to settle with increasing the compression ratio to mitigate minor connection issues in a manner that is almost unnoticeable to the user, and then only resorting to the omission of scan data to when a connection issue is no longer manageable simply through data compression.

It is noted that throughout this text omission of data and data compression have different meaning, although data compression can be interpreted as leaving out data. For the purpose of this disclosure, omitting data is meant as leaving out certain types of data, e.g. color data, whereas data compression is the process of encoding, restructuring, or otherwise modifying scan data in order to reduce its size.

The partial digital representation may have a lower resolution and/or color depth than the digital representation. This is advantageous as it leads to an overall reduction in latency by reducing the amount of data transmission in the downlink. It is therefore particularly advantageous if the connection issue can be isolated to pertaining to the downlink as the system/method may proceed by transmitting scan data as if it was in the full connection mode, while mitigating the downlink ussie by minimizing the amount of data coming back from the remote computing device.

The partial connection mode may comprise buffering and/or storing the part of the scan data that is not included in the reduced amount of the scan data as buffered scan data at the local device. The partial connection mode may comprise transmitting the buffered/stored scan data from the local device to the remote computing device after transmitting a reduced amount of the scan data, such as after scan data of the full dental object has been obtained. Such embodiments will have a delay transmission of the scan data that is not included in the reduced amount of the scan data, or to put it in other words, the parts of that scan data which are not essential to generate/update the digital representation or partial digital representation will be not be transmitted immediately but rather stored locally so as to free up connection bandwidth for the parts of the scan data that is necessary to generate/update the digital representation or partial digital representation. Through this, it will be possible to achieve a final 3D representation in full quality, albeit after the delayed transmission of the remaining scan data after the scan is complete.

The partial connection mode may comprise processing at least a part of the secondary scan data into locally processed data at the local device. The partial connection mode may comprise combining the received partial digital representation with the locally processed data, and displaying the partial digital representation with the locally processed data. At least part of the color data may be processed at the local device. Some applications of cloud assisted scanning still involve a dedicated software application to run on the local device, unlike applications run from an internet browser where the local device only needs an internet browser. In such applications, the method/system of the invention may leverage the local software to perform less computationally heavy processing. Thereby a hybrid system where the computationally heavy tasks, e.g. registering 3D geometry, may be done remotely, while the computationally lighter tasks may be performed locally, so that less data has to be transmitted between the local device and remote computing device.

Colors of the partial 3D representation may be predicted at the remote computing device. If color data is omitted in the reduced amount of the scan data, another approach may be to artificially generate color for the partial digital representation whereby a colored visualization may be displayed despite the omission of the color data. Predicting the colors of the partial 3D representation may be done by feeding the partial 3D representation and/or the reduced amount of scan data to a machine learning algorithm or a dental segmentation algorithm, to predict what type(s) of object(s) are present in the partial 3D representation, e.g. teeth, metal implants, or gingiva, and applying a preset color according to the predicted type(s) of object(s). This may reduce the load on the uplink as color data does not have to be transmitted during the scanning session, without reducing the user experience as the user may still see a colored partial 3D representation on their display.

The method may comprise, if the performance indicator is less than a critical performance threshold which is lower than the primary performance threshold, entering an offline mode, wherein the offline mode comprises:
- storing the scan data at the local device,
- at the local device, identifying which of the patient's teeth has been scanned based on the scan data, and
- displaying an indication of which of the patient's teeth has been scanned.

In the rare situations where the connection fails completely or almost completely, it is advantageous to have an offline mode as a fallback resort. In the offline mode, scan data will be stored at the local device, so that it may be transmitted to the remote computing device at a later point in time when the connection has been re-established. Identifying which of the patient's teeth has been scanned may be done by feeding the scan data or data based thereon to a machine learning algorithm or a dental segmentation algorithm, to predict which teeth have been covered by the scan data obtained thus far, e.g. upper left third molar, lower left cuspid, upper right central incisor, etc. Displaying an indication of which of the patient's teeth has been scanned may be done in a stored generic dental chart, e.g. by changing the color of scanned teeth to green, or a similar mock model of the human set of teeth.

Further disclosed is a scanning system comprising:
- an intraoral scanner configured for obtaining scan data of a dental object, wherein the scan data comprises primary scan data indicative of the 3D geometry of the dental object of a patient, wherein the scan data comprises secondary scan data indicative of one or more characteristics other than the 3D geometry of the dental object;
- a display;
- a connection module configured for establishing a connection between the intraoral scanner and the display and a remote computing device, and for obtaining a performance indicator by detecting a performance of the connection; and
- one or more processors configured for:
   - evaluating the performance indicator and based on the evaluation:
      if the performance indicator exceeds a primary performance threshold, entering a full connection mode, wherein the full connection mode comprises:
         - transmitting the scan data to the remote computing device,
         - receiving a digital representation of the dental object computed from the transmitted scan data from the remote computing device, and
         - displaying the digital representation on the display,
      if the performance indicator is less than the primary performance threshold entering a partial connection mode, wherein the partial connection mode comprises:
         - transmitting a reduced amount of the scan data to the remote computing device,
         - receiving a partial digital representation of the dental object computed from the transmitted scan data from the remote computing device, and
         - displaying the partial digital representation on the display.

The partial connection mode may comprise:
- generating the reduced amount of scan data by:
   ∘ compressing at least a part of the scan data,
   ∘ reducing a resolution of the primary scan data, and/or
   ∘ omitting at least part of the secondary scan data from the scan data.

Compressing at least a part of the scan data may be performed at a compression ratio determined based on the performance indicator.

The secondary scan data may comprise color data indicative of one or more colors of the dental object. Omitting at least part of the secondary scan data from the scan data may comprise reducing the color depth of the color data or omitting the color data. The partial 3D representation may be in a reduced color depth or monochrome.

The secondary scan data may comprise infrared, IR, data. Omitting at least part of the secondary scan data from the scan data comprises omitting the IR data. The secondary scan data may comprise voice data comprising voice recordings of a user recorded during the step of obtaining scan data of the dental object. Omitting at least part of the secondary scan data from the scan data may comprise omitting the voice data. The secondary scan data may comprise fluorescence data. Omitting at least part of the secondary scan data from the scan data may comprise omitting the fluorescence data.

The connection module may be configured for:
- transmitting time stamp data to the remote computing device, and
- receiving a result of a transmission time measurement computed from the transmitted time stamp data from the remote computing device.

The connection module may be configured for:
- transmitting a return data package to the remote computing device,
- receiving the return data package from the remote computing device, and
- calculating a round-trip time based on the time difference between when the return data package was transmitted and received at the local device.

The connection module may be configured for measuring a data packet loss rate of the connection. The connection module may be configured for measuring a data packet error rate of the connection.

To generate the reduced amount of scan data, the one or more processors may be configured for:
∘ if the performance indicator is between the primary performance threshold and a secondary performance threshold which is lower than the primary performance threshold, compressing at least a part of the scan data, preferably all of the scan data, at a dynamic compression ratio selected based on the performance indicator, and
∘ if the performance indicator is less than the secondary performance threshold, omitting at least a part of the secondary scan data from the scan.

The partial digital representation may have a lower resolution and/or color depth than the digital representation.

When in the partial connection mode, the one or more processors may be configured for buffering the part of the scan data that is not included in the reduced amount of the scan data as buffered scan data at the local device. When in the partial connection mode, the one or more processors may be configured for transmitting the buffered scan data from the local device to the remote computing device after transmitting a reduced amount of the scan data. When in the partial connection mode, the one or more processors may be configured for processing at least a part of the secondary scan data into locally processed data at the local device. When in the partial connection mode, the one or more processors may be configured for:
- combining the received partial digital representation with the locally processed data, and
- displaying the partial digital representation with the locally processed data.

When in the partial connection mode, the one or more processors may be configured for processing at least part of the color data. Color(s) of the partial 3D representation may be predicted at the remote computing device when in the partial connection mode. The one or more processors may be configured for:
if the performance indicator is less than a critical performance threshold which is lower than the primary performance threshold, entering an offline mode, wherein the offline mode comprises:
- storing the scan data at the local device,
- at the local device, identifying which of the patient's teeth has been scanned based on the scan data, and
- displaying an indication of which of the patient's teeth has been scanned.

Displaying an indication of which of the patient's teeth has been scanned may comprise displaying an image stored at the local device, the image comprising teeth corresponding to those of the patient's teeth has been scanned.

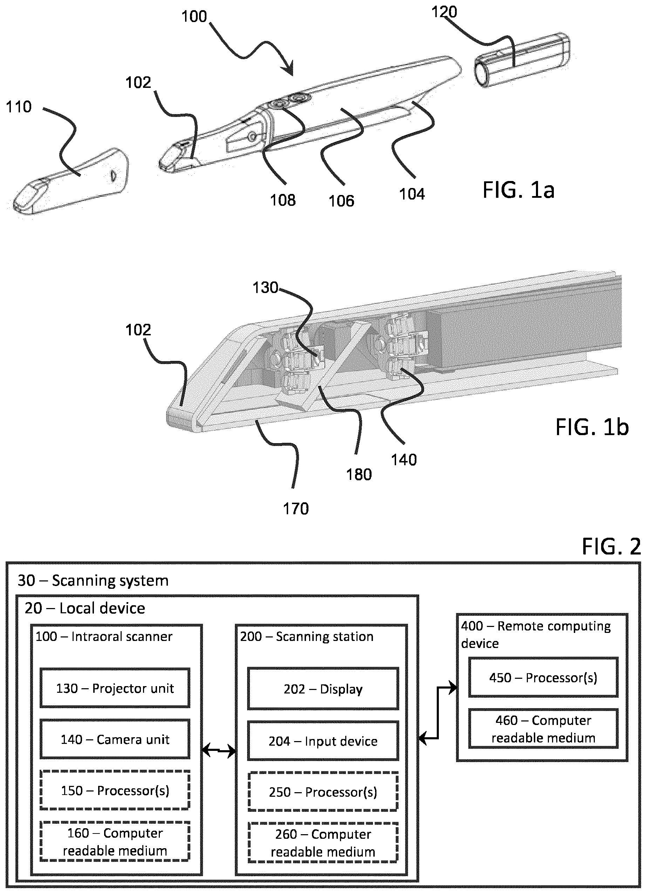

FIG. 1a illustrates an intraoral scanner 100 used in the scanning system 30 or method of the invention. The intraoral scanner 100 comprises a distal end 102 configured to be at least partially inserted into the patient's mouth. The distal end 102 comprises a scanner window 150 through which light can be projected onto the dental object 10 to be scanned and images of the illuminated object can be captured. The distal end 102 is further configured for having a sleeve 110 releasably attached over it. The sleeve 110 is provided as a hygienic barrier and may be changed between patients to avoid contamination between patients. The sleeve 110 comprises a sleeve window which, when the sleeve 110 is attached onto the distal end 102, overlaps at least partially with the scanner window 150.

The intraoral scanner 100 also comprises a proximal end 104 opposite the distal end 102. The proximal end 104 may comprise an opening configured for receiving and holding a battery 120, preferably a rechargeable battery, for powering the intraoral scanner 100. Alternatively, the battery 120 may be arranged inside the intraoral scanner 100, in which case the battery can be recharged through a power interface of the intraoral scanner 100. Between the distal end 102 and the proximal end 104, the intraoral scanner 100 comprises a scanner body 106 configured for being held by a user, e.g. a dentist or a dental technician. For the user to control the intraoral scanner 100 during scanning sessions, the intraoral scanner 100 comprises a scanner interface 108, which may comprise one or more push buttons and/or one or more touch interfaces.

FIG. 1b illustrates a cross-sectional view of an intraoral scanner 100 used in the scanning system 30 or method of the invention. The shown intraoral scanner 100 comprises multiple camera units 140, e.g. four or eight camera units 140, each configured for acquiring 2D images of the object 10. By having multiple camera units 140, the intraoral scanner 100 may acquire multiple 2D images substantially simultaneously. The shown intraoral scanner 100 comprises multiple projector units 130 configured for projecting light, e.g. patterned light, onto the object 10. Both the camera units 140 and the projector units 130 are arranged such that their optical axes are parallel or slightly angled to a longitudinal axis of the intraoral scanner 100. To allow the camera units 140 and the projector units 130 to view/illuminate the object 10, mirrors 180 are arranged in their optical path to direct their optical path towards the scanner window 170.

FIG. 2 illustrates a block diagram of a scanning system 30 of the invention or used for the method of the invention. The scanning system 30 comprises a local device 20 which can establish a connection 40 to a remote computing device 400. The local device 20 comprises an intraoral scanner 100 and a scanning station, also referred to as a local computing device, 200. The intraoral scanner 100 is configured for obtaining scan data of a dental object 10. The scan data comprises primary scan data indicative of the 3D geometry of the dental object 10 of a patient. The scan data comprises secondary scan data indicative of one or more characteristics other than the 3D geometry of the dental object 10. The local device 20 comprises one or more connection module(s) which are configured for transmitting the scan data to the remote computing device 400 which may then, from the scan data, generate a digital 3D representation of the scanned dental object 10 and return a digital representation of the dental object 10 to the local device. The digital representation returned by the remote computing device 400 may be in 3D or it may be a 2D model of the digital 3D representation. The scanning station 200 comprises a display 202 configured for displaying the digital representation received from the remote computing device 400. This may assist the user in identifying which parts of the dental object 10 they have already scanned and which they still need to scan and move the intraoral scanner 100 in a position where they may scan the missing parts.

The scanning system 30 may comprise multiple processors, at least some of which will be comprised by the remote computing device 400 so that it can generate the digital 3D representation and the digital representation. One or more processors may be comprised by the local device 20, such as in the intraoral scanner 100 and/or in the scanning station. Computing the digital representation of the dental object and the partial digital representation of the dental object is thus performed at the processor(s) 450 of the remote computing device 400, while the remaining steps may be executed by one or more of the processor(s) 450 of the remote computing device 400, the processor(s) 150 of the intraoral scanner 100, and/or the processor(s) 250 of the scanning station 200.

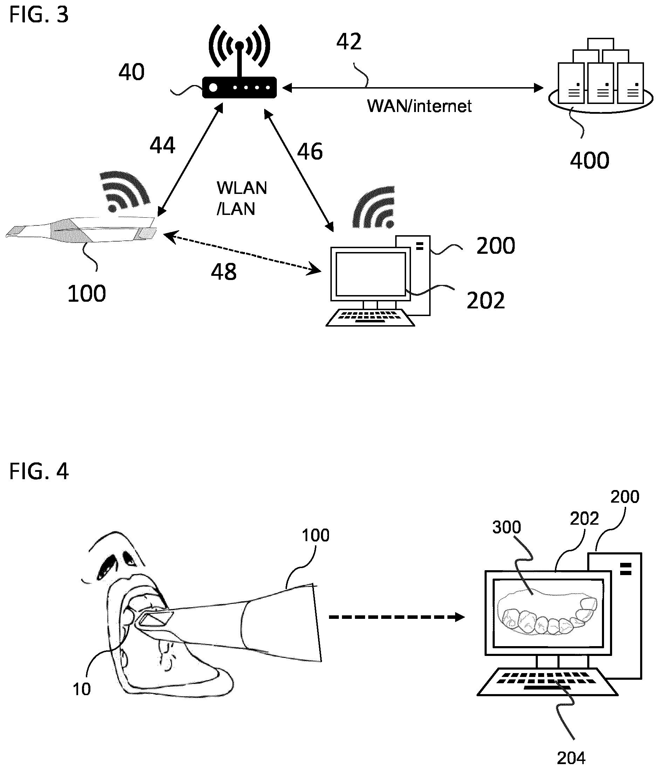

FIG. 3 shows an example of a scanning system of the invention. In the shown example, the intraoral scanner 100 and the scanning station 200 are both wireless devices coupled to a LAN network through one or more router(s) 40. Multiple configurations for the Lan connections fall under the invention. In the shown embodiment, the intraoral scanner 100 and the scanning station 200 each have their own LAN connections 44, 46, while a direct connection 48 between the two is optional as they may communicate through the router(s) 40. Alternatively, one of the intraoral scanner 100 or scanning station 200 may act as a relay for the other, in which case a direct connection 48, be it wired or wireless, between the intraoral scanner 100 and scanning station 200 is necessary while the connection 44, 46 of the device being relayed may be omitted.

To connect the local device 20 to the remote computing device 400, the connection further comprises a Wide Area Network (WAN) connection 42, such as the internet. Scan data may thus be transmitted from the intraoral scanner 100 to the remote computing device 400 via the LAN connection 44 of the intraoral scanner 100 and the WAN connection 42, and the remote computing device 400 may in turn transmit a digital representation or partial digital representation based on the scan data to the scanning station 200 via the WAN connection 42 and the LAN connection 46 of the scanning station 200.

FIG. 4 shows an illustration of a scanning session at the local device 20. To obtain scan data, the user, e.g. the dentist or dental technician, inserts the intraoral scanner 100 into the mouth of a patient to acquire 2D images of the dental object 10. The scan data, or reduced amount of scan data, is then transmitted to the remote computing device 400 which returns a digital representation 300 or partial digital representation 300 as explained in FIG. 3. From the local device 20 perspective, the user may in substantial real-time see the digital representation 300 or partial digital representation 300 corresponding to the scan data that has just been acquired on the display 202 of the scanning station 200, despite the scan data having been processed remotely. By displaying the digital representation 300 or partial digital representation 300, the user may see the progress of the scanning session by observing the digital representation 300 or partial digital representation 300 representing the scan data collected thus far.

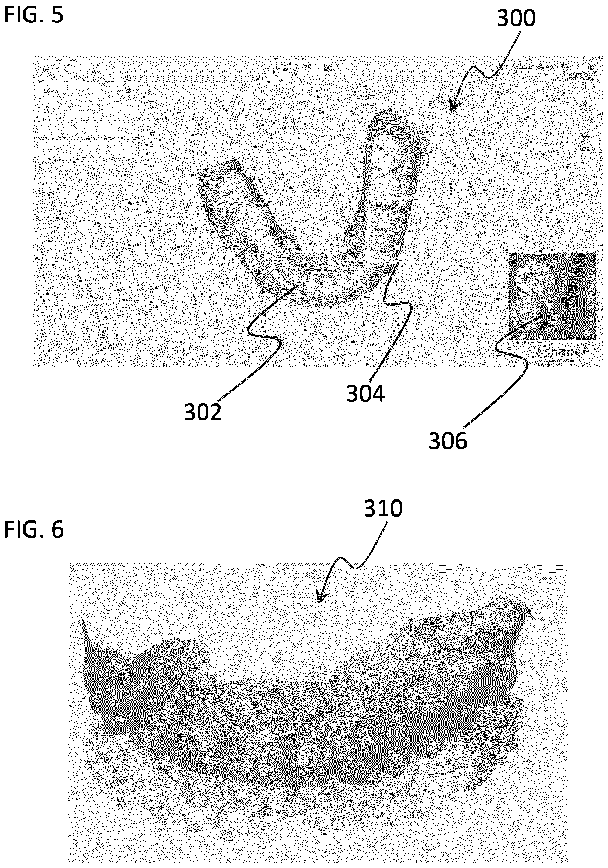

FIG. 5 shows an example of a GUI comprising a digital representation or partial digital representation 300 as it will appear on the display 202 at the local device 20. The digital representation/partial digital representation 300 may comprise a 3D visualization 302 representing the 3D geometry of the parts of the dental object 10 that have been scanned up to the current point of the scanning session. The digital representation/partial digital representation 300 may comprise a scan box 304 overlaid on top of the 3D visualization 302 to indicate the intraoral scanner's current field of view in relation to the dental object 10. The digital representation/partial digital representation 300 may additionally or alternatively comprise a viewfinder image 306 showing a view of the intraoral scanner's current field of view. The viewfinder image may be generated at the local device and overlaid the digital representation/partial digital representation 300 before displaying the combined viewfinder image 306 and digital representation/partial digital representation 300.

The 3D visualization 302 and/or viewfinder image 306 may be presented in color or in monochrome depending on whether the system is operating in the full connection mode or in the partial connection mode. In the partial connection mode, secondary scan data, or parts thereof, may be omitted or reduced in size and quality when generating the reduced amount of scan data, which may affect the quality of the coloring of the partial digital representation 300. When faced with severe connection issues, it may be necessary to fully omit color data when generating the reduced amount of scan data, which in turn will lead to the remote computing device 400 computing a monochrome partial digital representation 300. While this may affect the user's experience, it will also allow them to proceed with the scanning session despite the connection issues.

Various embodiments may add color to the partial digital representation artificially. Color(s) may be added at the remote computing device 400 by predicting the type of material, or materials, are present in the scan data, and add color(s) accordingly, e.g. add eggshell white to teeth, light pink the gingiva, and/or metallic color to visible implants. If the local device 20 is running a software application dedicated to intraoral scanning, rather than an internet browser, some processing may also take place locally. In this case, color(s) may either be artificially created as explained above, or color(s) may be computed based on the secondary scan data, e.g. the parts of the secondary scan data that was missing from the reduced amount of scan data, and the locally computed color(s) may then be added to the partial digital representation received from the remote computing device 400 before being displayed.

Additionally or alternatively to reducing/removing color(s) in the partial connection mode, other measures may be taken to reduce the amount of data being transferred back and forth between the local device 20 and the remote computing device 400. The resolution of the primary scan data may be reduced, thereby also leading to a reduction in the resolution of the partial digital representation. If the connection issue can be isolated to the downlink, then the partial digital representation may be generated at a resolution lower than that of the digital representation and/or the refresh rate of the display may be reduced, thereby requiring less frequent updates of the partial digital representation 300.

FIG. 6 shows an example of the completed final 3D representation 310 of a dental object 10 being a full set of human teeth. The final 3D representation 310 comprises a point cloud, as shown in the figure, and/or a mesh, e.g. a mesh formed by triangles. While it is the final objective of the scanning session to obtain a final 3D representation 310 that meets the quality requirements of a forthcoming dental procedure, e.g. a crown or dental bridge procedure, the digital representation and partial digital representation being displayed as the scanning session progress does not need to meet these requirements as they merely serve to guide the user through the scanning session. The partial connection mode may therefore comprise buffering/storing parts of the scan data for later transmission, e.g. after the scanning session when the connection issue has been resolved, whereby a final 3D representation 310 in higher quality than the partial digital representation may eventually be obtained based on scan data collected during the scanning session.

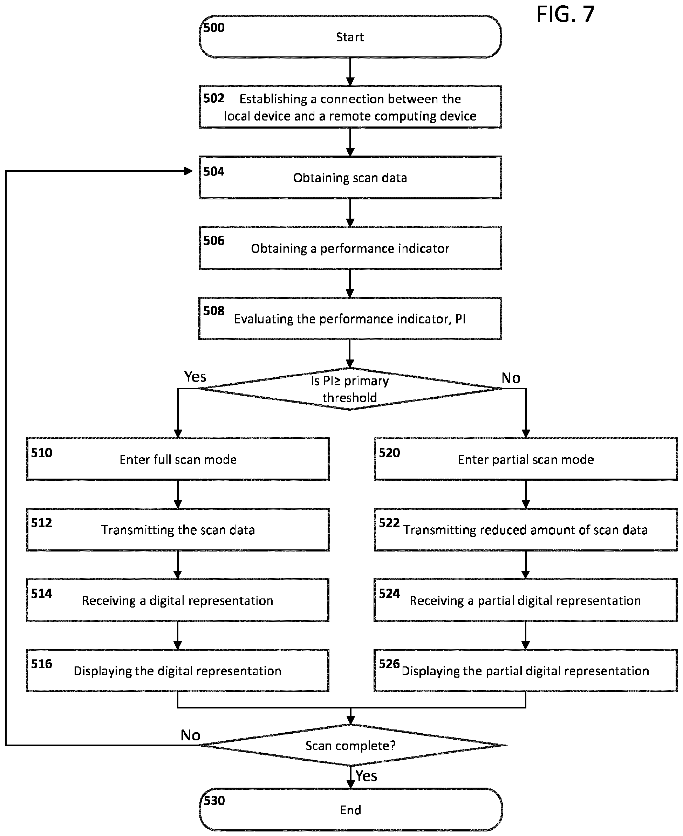

FIG. 7 shows a flowchart of a computer implemented method of the invention from the local device's perspective. The method is started/initiated 500 by the user activating the local device 20. The local device 20 may then establish 502 the connection between the local device 20 and the remote computing device 400, e.g. by sending a request to establish the real-time interactive scanning session comprising device identification (ID) of the local device. In response to a request to establish the real-time interactive scanning session, the remote computing system 400 may determine a device capability, e.g. hardware and/or software capabilities, of the requesting local device 20, e.g. the phone, tablet, or laptop comprised by the local device 20, a connection capability, e.g. bandwidth, latency and/or error rate, of network connection connecting the local and remote computing devices 20, 400, and one or more target quality parameters of the scanning session (e.g., resolution of the output video stream(s), scanning response latency, etc.), and accordingly, associates one of its virtual machines with the real-time interactive session for establishing the session. After having established the connection, a visible and/or audible output transducer at the local device 20 may indicate that a connection with the remote computing device is active, e.g. a colored LED on the intraoral scanner may turn green and/or a visual indicator in the GUI on the display 202 may inform the user that a connection has been established.

The user may then start to obtain 504 the scan data. Obtaining 504 the scan data may be performed as previously explained by acquiring 2D images inside the patient's mouth using the intraoral scanner 100. The 2D images may form part of the scan data or they may be preprocessed, e.g. in the intraoral scanner 100 into the scan data. Preprocessing at the local device 20 may comprise extracting 3D geometry from the 3D images of a subscan. In addition to 2D images for 3D data, other types of 2D images may also be acquired during the course of a subscan. IR and fluorescence data may be collected during separate image frames from the image frames used for acquiring 2D images for 3D data, or in the same image frames using one or more image sensors and/or camera pixels dedicated for IR or fluorescent wavelengths of light. Other types of scan data may be recorded using other sensors than the camera unit(s) 140. Voice data may be recorded using a microphone in the intraoral scanner 100 and/or scanning station 200, and the voice data may be linked to the other parts of the scan data, e.g. through time stamping, so that the user can replay their verbal notes if inspecting the final 3D representation at a later date. The scan data may then be separated into primary scan data, i.e. the part of the scan data comprising the 3D geometry or needed to extract the 3D geometry of the dental object 10, and the secondary scan data, i.e. the part of the scan data that is indicative of characteristic(s) other than the 3D geometry of the dental object.

Before transmitting the scan data, the method comprises obtaining 506 a performance indicator (PI) by detecting a performance of the connection. Detecting the performance of the connection may comprise detecting an RTT latency time, e.g. by sending a ping from either the local device 20 or the remote computing device 400 and detecting the echo of the ping by the same device. During a scanning session, detecting the performance of the connection may be based on previous transmissions of scan data and/or digital representations or partial digital representations, e.g. error detection in data packages and/or transmission time.

Having obtained 506 the PI, the method comprises evaluating 508 the PI by comparing the PI to a primary performance threshold. The primary performance threshold may be a preset parameter, or it may be dynamically set based on the rate at which scan data is generated. The units of the primary performance threshold will depend on the type(s) of connection parameters the PI represents. As an example, if the PI is a measure of the current bandwidth, the primary performance threshold may be set according to a sealing of scan data acquisition. For intraoral scanners such as the TRIOS series made by the applicant, a required bandwidth for transferring the full amount of scan data generated would be about 41 mbit/s. The primary performance threshold may be set accordingly, optionally with the addition of a margin for safety, as 50 mbit/s. Again, it is noted that the required bandwidth depends on the type, i.e. brand and model, of the intraoral scanner

If the evaluation 508 of the PI determines that the PI is at or above the primary performance threshold, i.e. the connection meets the requirements for regular cloud scanning, the method comprises entering 510 a full scan mode. In the full connection mode, the scan data may be uncompressed or subjected to lossless compression so that no significant information is lost. Next, the scan data is transmitted 512 to the remote computing device 400 which will compute a digital representation 300 based on the scan data. In the full connection mode, computing the digital representation 300 may comprise calculating the 3D geometry of the scanned object from the primary scan data and/or registering the 3D geometry of the scan data the remote computing device 400 just received to the 3D geometry of scan data previously received by the remote computing device 400. Calculating the 3D geometry may result in a three-dimensional point cloud representing the surface of the parts of the dental object 10 in the scan data the remote computing device 400 just received. Registering the 3D geometry can be done in several ways, one is to use an Iterative Closest Point (ICP) algorithm which minimizes the difference between points of the point cloud resulting from the data that was just received and the points of the point cloud resulting from the accumulated previous scan data, i.e. the scan data the remote computing device have received thus far in the current scanning session.

In the full connection mode, computing the digital representation 300 may further comprise computing characteristics other than the 3D geometry based on the secondary scan data. A first other characteristic may be color(s) which can be calculated based on color data comprised by the secondary scan data. During the scanning session, adding colors to the digital representation increases the user experience as the user may see a digital representation of their scan progress in natural colors. More importantly, colors are important for the final 3D representation 310 as it allows dental prosthetics designed based on the final 3D representation 310 to be color matched to adjacent teeth resulting in teeth that appear more natural for the patient after the dental procedure. Another characteristic may be diagnostic information which can be calculated based on diagnostics data, i.e. IR and/or fluorescence data comprised by the secondary scan data. Diagnostic information may aid the user in identifying dental conditions such as carries and plaque in the patient.

After the computation of the digital representation 300 by the remote computing device 400, the method comprises receiving 514 the digital representation at the local device 20. After receiving 514 the digital representation, the local device 20 proceeds by displaying 516 the digital representation whereby the user may overserve the progress of the scanning session by seeing a digital representation based on the scan data accumulated thus far in the scanning session.

If the evaluation 508 of the PI determines that the PI is below the primary performance threshold, i.e. the connection fails to meet the requirements for regular cloud scanning, the method comprises entering 522 a partial scan mode. In the partial connection mode, the scan data may be compressed, preferably at a dynamic compression ratio based on the PI, or if compression is insufficient, i.e. if the connection issue is severe, the secondary scan data may be at least partially omitted, whereby a reduced amount of scan data is generated. For minor connection issues, i.e. if the PI is below the primary performance threshold and above a secondary performance threshold, it may also be viable to use a first compression ratio, e.g. lossless compression, for the primary scan data and a second compression ratio for the secondary scan data, wherein the secondary compression ratio is higher than the first compression ratio. In the full connection mode, the primary and/or secondary scan data may be compressed using High Efficiency Video Coding (HEVC), also known as H.265. HEVC encoders can trade off computational complexity, compression rate, robustness to errors, and encoding delay time.

Next, the reduced amount of scan data is transmitted 522 to the remote computing device 400 which will compute a partial digital representation based on the reduced amount of scan data. In the partial connection mode, computing the partial digital representation may comprise calculating the 3D geometry of the scanned object from the primary scan data and/or registering the 3D geometry of the reduced amount of scan data the remote computing device 400 just received to the 3D geometry of reduced amount of scan data and/or scan data previously received by the remote computing device 400. It is noted that the 3D geometry extracted in the partial connection mode may be registered to the 3D geometry extracted in the full connection mode and vice versa, so that the system/method may switch between the two connection modes during a scanning session in case the connection performance changes during the course of the scanning session.

While the secondary scan data may be omitted or compressed in the partial connection mode, the other characteristicsadded this on page 16 may still be calculated. Colors may be calculated based on the color data of the secondary data, if compression rather than omission has been used, although this may result in a lower color depth than would be achievable in the full connection mode. If color data has been omitted in the reduced amount of scan data, color(s) may be predicted as described above. If a change from full connection mode to partial connection mode happens during the scanning session, predicting the color(s) may be based on secondary scan data received earlier in the scanning session while in the full connection mode, e.g. by assuming that color of the patients teeth are homogenous and using a color calculated while in the full connection mode as basis for artificially applying colors in the partial connection mode.

After the computation of the partial digital representation by the remote computing device 400, the method comprises receiving 524 the partial digital representation at the local device 20. After receiving 524 the partial digital representation, the local device 20 proceeds by displaying 526 the partial digital representation whereby the user may overserve the progress of the scanning session by seeing a digital representation based on the scan data accumulated thus far in the scanning session.

Regardless of whether the system/method is in the full connection mode or partial connection mode, it is then determined, e.g. by the user, whether the dental object 10 has been scanned. If the scan is not complete, the method proceeds by repeating the steps from obtaining 504 scan data. The determination of whether the scan is complete may be assumed to be no, i.e. the scan is not complete, until an input for ending or pausing the scan is detected, such as the press of a button or touch sensitive surface on the intraoral scanner 100 associated with ending the scan. When the scan is completed, the scanning session may be ended 530.

When the scan is completed, the method may comprise transmitting parts of the reduced amount of scan data buffered or stored on the local device, which were not transmitted as the scan was ongoing. Having obtained sufficient scan data to create a final 3D representation 310 of the dental object 10, the remote computing device may perform post processing to refine and/or optimize the final 3D representation 310. Transmitting buffered/stored data and postprocessing may be done at a later time, such as when the connection issues have been resolved.

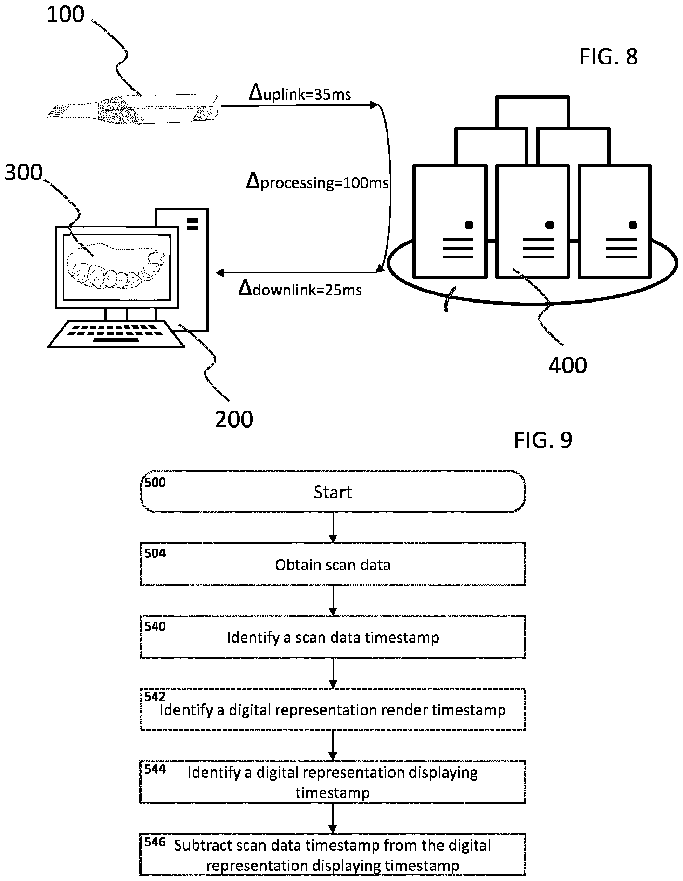

FIG. 8 illustrates an example of RTT latency from the time scan data is obtained to the time a digital representation or partial digital representation is displayed. As previously noted, the perceivable metric of the connection performance to the user is the time it takes between them performing an action, such as hovering the intraoral scanner 100 over a part of the dental object, and the expected result of that action, such as them seeing that part of the dental object added to the displayed digital representation or partial digital representation. The RTT latency is the accumulated time of all the process steps between the action and the result. For a cloud scanning scenario, this may be the time it takes to transmit the scan data or reduced scan data, Δᵤₚₗᵢₙₖ, the time it takes for the remote computing device to compute the digital representation or partial digital representation, Δ_{processing}, and the time it takes to transmit the digital representation or partial digital representation back to the local device 20, Δ_{downlink}, combined. In the example shown in FIG. 8, the RTT is therefore 30ms (Δᵤₚₗᵢₙₖ) plus 100ms (Δ_{processing}) plus 25ms (Δ_{downlink}) resulting in an RTT latency of 155ms.

FIG. 9 shows a flowchart of an RTT latency measurement that may form part of obtaining 506 the performance indicator. After obtaining 502 the scan data, a scan data timestamp may be identified 540, wherein the scan data timestamp indicates the time at which the scan data was obtained. Then, when performing the step of displaying 516, 526 the digital representation, the method comprises Identifying 542 a digital representation displaying timestamp, wherein the digital representation displaying timestamp indicates the time at which the digital representation or partial digital representation was displayed at the local device 20. The RTT latency may then be determined by subtracting 546 the scan data timestamp from the digital representation displaying timestamp, whereby a value for the time between action of obtaining scan data and the result of that action, i.e. the displaying of the digital representation generated from that scan data, is obtained.

Optionally, the method may also comprise identifying 544 a digital representation render timestamp, wherein the digital representation render timestamp indicates the time at which rendering of the digital representation or partial digital representation was started and/or completed at the remote device 400. Thereby, the individual contributors to the RTT latency may be determined, i.e. Δᵤₚₗᵢₙₖ= digital representation render (start) timestamp - scan data timestamp, Δ_{processing}= digital representation render (end) timestamp - digital representation render (start) timestamp, and Δ_{downlink}= digital representation displaying timestamp - digital representation render (end) timestamp.

In some implementations, the remote computing device 400 measures network latency between the local device 20 and the remote computing device 400. If the measured latency is above a latency threshold and a lower-latency connection is available, e.g. a cellular 5g connection or ethernet, the remote computing device 400 can suggest that the local device 20 changes to the lower latency connection, or invite a user of the local device 20 to change the local device 20 to the lower latency connection. For example, if the local device 20 is on a cellular wireless connection, and a local network is available, the remote computing device 400 can suggest that the local device 20 should connect through the available local network.

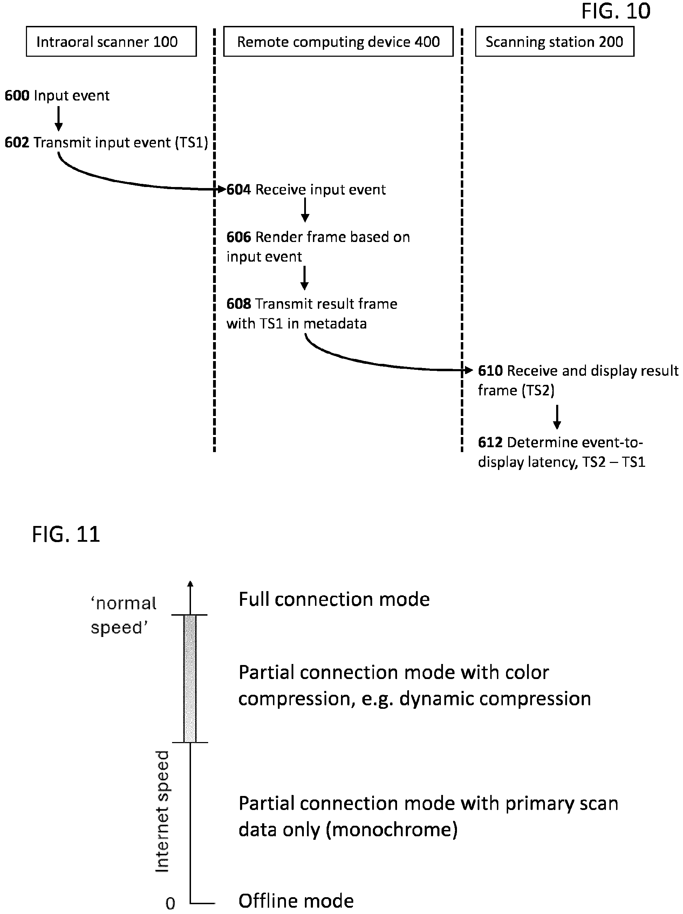

FIG. 10 illustrates a process for event-to-display latency measurements, similar to that described in FIG. 9, which may be used in the step of obtaining 506 the performance indicator. The measurement is started by an input event 600, such as scan data being obtained or a user interaction with a button on the intraoral scanner 100 of the local device 20. The input event or data based on the input event is be timestamped, TS1, and transmitted to the remote computing device 400. The remote computing device 400 then receives 604 the input event or the data based on the input event, and proceeds to render 606 a display frame based on the input event or the data based on the input event. The remote computing device 400 then transmits 608 the resulting display frame including TS1 in the metadata to the scanning station 200 of the local device 20. The scanning station 200 then receives and displays 610 the resulting display frame and generates a timestamp, TS2. Finally, the scanning station may determine 612 an event-to-display latency by subtracting TS1 from TS2.

FIG. 11 illustrates an embodiment where the measures taken in the partial connection mode increases as the connection performance declines. Above the primary performance threshold, denoted 'normal speed', the method/system runs in full connection mode.

Below the primary performance threshold and above a secondary performance threshold, the method/system runs in a first partial connection mode in which color data is compressed at a ratio higher than the lossless compression that may be used in the full connection mode when generating the reduced amount of scan data. In the first partial connection mode, color compression may be performed using dynamic data compression, by using a compression ratio depending on the performance indicator or the performance of the connection. Put in order words, the worse the connection is the more the color data is compressed. The compression used for the color data, or the secondary scan data, while in the partial connection mode may be lossy compression, i.e. compression leading to irreversible loss of information.

Below the secondary performance threshold, the method/system runs in a second partial connection mode in which color data is omitted when generating the reduced amount of scan data. While this may result in a monochromatic partial digital representation, it may cut the bandwidth requirements by about 50% meaning that the user can complete a scanning session even when the connection is performing significantly lower than the requirements for full connection mode.

The system/method may optionally also comprise an offline mode which may be entered upon complete failure of the connection, or optionally below a tertiary performance threshold lower than the secondary performance threshold. In the offline mode, the system/method may resort to storing the obtained scan data at the local device 20 and using either a machine learning algorithm or segmentation algorithm run on the local device 20 to predict which teeth has been scanned and present a locally stored mock model of a set of human teeth, e.g. a dental chart, in which the teeth determined to have been scanned are indicated as having been scanned, e.g. by changing their color in the mock model to green.

### Further details

Embodiments of the invention are disclosed in the following list of enumerated items:
1. A computer-implemented method for facilitating 3D intraoral scanning using cloud computing, the method comprising:
   - obtaining scan data of a dental object at a local device, wherein the scan data comprises primary scan data indicative of the 3D geometry of the dental object of a patient, wherein the scan data comprises secondary scan data indicative of one or more characteristics other than the 3D geometry of the dental object;
   - establishing a connection between the local device and a remote computing device;
   - obtaining a performance indicator by detecting a performance of the connection;
   - evaluating the performance indicator and based on the evaluation:
      if the performance indicator exceeds a primary performance threshold, entering a full connection mode, wherein the full connection mode comprises:
         - transmitting the scan data from the local device to the remote computing device,
         - receiving, at the local device from the remote computing device, a digital representation of the dental object computed from the transmitted scan data, and
         - displaying the digital representation,
      if the performance indicator is less than the primary performance threshold entering a partial connection mode, wherein the partial connection mode comprises:
         - transmitting a reduced amount of the scan data from the local device to the remote computing device,
         - receiving, at the local device from the remote computing device, a partial digital representation of the dental object computed from the transmitted scan data, and
         - displaying the partial digital representation.
2. The method of item 1, wherein the full connection mode comprises:
   - by the remote computing device, computing the digital representation of the dental object.
3. The method of items 1 or 2, wherein the partial connection mode comprises:
   - by the remote computing device, computing the partial digital representation of the dental object.
4. The method of item 1, wherein the partial connection mode comprises:
   - generating the reduced amount of scan data by:
      ∘ compressing at least a part of the scan data,
      ∘ reducing a resolution of the primary scan data, and/or
      ∘ omitting at least part of the secondary scan data from the scan data.
5. The method of item 4, wherein compressing at least a part of the scan data is performed at a compression ratio determined based on the performance indicator.
6. The method of any of the previous items, wherein the secondary scan data comprises color data indicative of one or more colors of the dental object.
7. The method of items 4 and 6, wherein omitting at least part of the secondary scan data from the scan data comprises reducing the color depth of the color data or omitting the color data.
8. The method of item 7, wherein the partial 3D representation is in a reduced color depth or monochrome.
9. The method of any of the previous items, wherein the secondary scan data comprises infrared (IR) data.
10. The method of items 4 and 9, wherein omitting at least part of the secondary scan data from the scan data comprises omitting the IR data.
11. The method of any of the previous items, wherein the secondary scan data comprises voice data comprising voice recordings of a user recorded during the step of obtaining scan data of the dental object.
12. The method of items 4 and 11, wherein omitting at least part of the secondary scan data from the scan data comprises omitting the voice data.
13. The method of any of the previous items, wherein the secondary scan data comprises fluorescence data.
14. The method of items 4 and 13, wherein omitting at least part of the secondary scan data from the scan data comprises omitting the fluorescence data.
15. The method of any of the previous items, wherein detecting the performance of the connection comprises:
   - transmitting time stamp data from the local device to the remote computing device, and
   - receiving a result of transmission time measurement computed from the transmitted time stamp data from the remote computing device.
16. The method of any of the previous items, wherein detecting the performance of the connection comprises:
   - transmitting a return data package from the local device to the remote computing device,
   - at the local device, receiving the return data package from the remote computing device, and
   - calculating a round-trip time based on the time difference between when the return data package was transmitted and received at the local device.
17. The method of any of the previous items, wherein detecting the performance of the connection comprises:
   - measuring a data packet loss rate between the local device and the remote computing device.
18. The method of any of the previous items, wherein detecting the performance of the connection comprises:
   - measuring a data packet error rate between the local device and the remote computing device.
19. The method of any of the previous items, wherein the local device comprises an intraoral scanner and a display.
20. The method of claim 19, wherein obtaining scan data of a dental object is performed using an intraoral scanner.
21. The method of items 19 or 20, wherein displaying the digital representation is performed using the display.
22. The method of any of items 19-21, wherein displaying the partial digital representation is performed using the display.
23. The method of any of the previous items and item 4, wherein generating the reduced amount of scan data comprises:
   ∘ if the performance indicator is between the primary performance threshold and a secondary performance threshold which is lower than the primary performance threshold, compressing at least a part of the scan data, preferably all of the scan data, at a dynamic compression ratio selected based on the performance indicator, and
   ∘ if the performance indicator is less than the secondary performance threshold, omitting at least a part of the secondary scan data from the scan.
24. The method of any of the previous items, wherein the partial digital representation has a lower resolution and/or color depth than the digital representation.
25. The method of any of the previous items, wherein the partial connection mode comprises:
   - buffering the part of the scan data that is not included in the reduced amount of the scan data as buffered scan data at the local device.
26. The method of item 25, wherein the partial connection mode comprises:
   - transmitting the buffered scan data from the local device to the remote computing device after transmitting a reduced amount of the scan data.
27. The method of any of the previous items, wherein the partial connection mode comprises:
   - processing at least a part of the secondary scan data into locally processed data at the local device.
28. The method of item 27, wherein the partial connection mode comprises:
   - combining the received partial digital representation with the locally processed data, and
   - displaying the partial digital representation with the locally processed data.
29. The method of items 6 and 27, wherein at least part of the color data is processed at the local device.
30. The method of any of the previous items and item 7, wherein colors of the partial 3D representation is predicted at the remote computing device.
31. The method of any of the previous items, wherein:
   if the performance indicator is less than a critical performance threshold which is lower than the primary performance threshold, entering an offline mode, wherein the offline mode comprises:
   - storing the scan data at the local device,
   - at the local device, identifying which of the patient's teeth has been scanned based on the scan data, and
   - displaying an indication of which of the patient's teeth has been scanned.
32. The method of item 31, wherein displaying an indication of which of the patient's teeth has been scanned comprises displaying an image stored at the local device, the image comprising teeth corresponding to those of the patient's teeth has been scanned.
33. The method of any of the previous items, wherein the steps are repeated continuously until the dental object has been fully scanned.
34. A computer program product comprising instructions which, when the program is executed by a computer, causes the computer to carry out the method of any of the previous items.
35. A non-transitory computer readable medium comprising instructions which, when executed by a computer, cause the computer to carry out the method of any of items 1-33.
36. A scanning system comprising:
   - an intraoral scanner configured for obtaining scan data of a dental object, wherein the scan data comprises primary scan data indicative of the 3D geometry of the dental object of a patient, wherein the scan data comprises secondary scan data indicative of one or more characteristics other than the 3D geometry of the dental object;
   - a display;
   - a connection module configured for establishing a connection between the intraoral scanner and the display and a remote computing device, and for obtaining a performance indicator by detecting a performance of the connection; and
   - one or more processors configured for:
      - evaluating the performance indicator and based on the evaluation:
         if the performance indicator exceeds a primary performance threshold, entering a full connection mode, wherein the full connection mode comprises:
            - transmitting the scan data to the remote computing device,
            - receiving a digital representation of the dental object computed from the transmitted scan data from the remote computing device, and
            - displaying the digital representation on the display,
         if the performance indicator is less than the primary performance threshold entering a partial connection mode, wherein the partial connection mode comprises:
            - transmitting a reduced amount of the scan data to the remote computing device,
            - receiving a partial digital representation of the dental object computed from the transmitted scan data from the remote computing device, and
            - displaying the partial digital representation on the display.
37. The scanning system of item 36, wherein, when in the partial connection mode, the one or more processors is/are configured for:
   - generating the reduced amount of scan data by:
      ∘ compressing at least a part of the scan data,
      ∘ reducing a resolution of the primary scan data, and/or
      ∘ omitting at least part of the secondary scan data from the scan data.
38. The scanning system of item 37, wherein compressing at least a part of the scan data is performed at a compression ratio determined based on the performance indicator.
39. The scanning system of any of items 36-37, wherein the secondary scan data comprises color data indicative of one or more colors of the dental object.
40. The scanning system of items 37 and 39, wherein omitting at least part of the secondary scan data from the scan data comprises reducing the color depth of the color data or omitting the color data.
41. The scanning system of item 40, wherein the partial 3D representation is in a reduced color depth or monochrome.
42. The scanning system of any of items 36-41, wherein the secondary scan data comprises infrared, IR, data.
43. The scanning system of items 37 and 42, wherein omitting at least part of the secondary scan data from the scan data comprises omitting the IR data.
44. The scanning system of any of the previous items, wherein the secondary scan data comprises voice data comprising voice recordings of a user recorded during the step of obtaining scan data of the dental object.
45. The scanning system of items 37 and 44, wherein omitting at least part of the secondary scan data from the scan data comprises omitting the voice data.
46. The scanning system of any of the previous items, wherein the secondary scan data comprises fluorescence data.
47. The scanning system of items 37 and 46, wherein omitting at least part of the secondary scan data from the scan data comprises omitting the fluorescence data.
48. The scanning system of any of items 36-47, wherein the connection module is configured for:
   - transmitting time stamp data to the remote computing device, and
   - receiving a result of a transmission time measurement computed from the transmitted time stamp data from the remote computing device.
49. The scanning system of any of items 36-48, wherein the connection module is configured for:
   - transmitting a return data package to the remote computing device,
   - receiving the return data package from the remote computing device, and
   - calculating a round-trip time based on the time difference between when the return data package was transmitted and received at the local device.
50. The scanning system of any of items 36-49, wherein the connection module is configured for:
   - measuring a data packet loss rate of the connection.
51. The scanning system of any of items 36-50, wherein the connection module is configured for:
   - measuring a data packet error rate of the connection.
52. The scanning system of any of items 36-51 and item 37, wherein, to generate the reduced amount of scan data, the one or more processors is/are configured for:
   ∘ if the performance indicator is between the primary performance threshold and a secondary performance threshold which is lower than the primary performance threshold, compressing at least a part of the scan data, preferably all of the scan data, at a dynamic compression ratio selected based on the performance indicator, and
   ∘ if the performance indicator is less than the secondary performance threshold, omitting at least a part of the secondary scan data from the scan.
53. The scanning system of any of items 36-52, wherein the partial digital representation has a lower resolution and/or color depth than the digital representation.
54. The scanning system of any of items 36-53, wherein, when in the partial connection mode, the one or more processors is/are configured for:
   - buffering the part of the scan data that is not included in the reduced amount of the scan data as buffered scan data at the local device.
55. The scanning system of item 25, wherein, when in the partial connection mode, the one or more processors is/are configured for:
   - transmitting the buffered scan data from the local device to the remote computing device after transmitting a reduced amount of the scan data.
56. The scanning system of any of the previous items, wherein, when in the partial connection mode, the one or more processors is/are configured for:
   - processing at least a part of the secondary scan data into locally processed data at the local device.
57. The scanning system of item 27, wherein, when in the partial connection mode, the one or more processors is/are configured for:
   - combining the received partial digital representation with the locally processed data, and
   - displaying the partial digital representation with the locally processed data.
58. The scanning system of items 6 and 27, wherein, when in the partial connection mode, the one or more processors is/are configured for processing at least part of the color data.
59. The scanning system of any of the previous items and item 7, wherein color(s) of the partial 3D representation is/are predicted at the remote computing device.
60. The scanning system of any of the previous items, wherein the one or more processors is/are configured for:
   if the performance indicator is less than a critical performance threshold which is lower than the primary performance threshold, entering an offline mode, wherein the offline mode comprises:
   - storing the scan data at the local device,
   - at the local device, identifying which of the patient's teeth has been scanned based on the scan data, and
   - displaying an indication of which of the patient's teeth has been scanned.
61. The scanning system of item 31, wherein displaying an indication of which of the patient's teeth has been scanned comprises displaying an image stored at the local device, the image comprising teeth corresponding to those of the patient's teeth has been scanned.

The use of the terms "first", "second", "third" and "fourth", "primary", "secondary", "tertiary" etc. does not imply any particular order, but are included to identify individual elements. Moreover, the use of the terms "first", "second", "third" and "fourth", "primary", "secondary", "tertiary" etc. does not denote any order or importance, but rather the terms "first", "second", "third" and "fourth", "primary", "secondary", "tertiary" etc. are used to distinguish one element from another. Note that the words "first", "second", "third" and "fourth", "primary", "secondary", "tertiary" etc. are used here and elsewhere for labelling purposes only and are not intended to denote any specific spatial or temporal ordering. Furthermore, the labelling of a first element does not imply the presence of a second element and vice versa.

It is to be noted that the word "comprising" does not necessarily exclude the presence of other elements or steps than those listed. It is to be noted that the words "a" or "an" preceding an element do not exclude the presence of a plurality of such elements.

It should further be noted that any reference signs do not limit the scope of the claims, that the exemplary embodiments may be implemented at least in part by means of both hardware and software, and that several "means", "units" or "devices" may be represented by the same item of hardware.

It should be appreciated that reference throughout this specification to "one embodiment" or "an embodiment" or "an aspect" or features included as "may" means that a particular feature, structure or characteristic described in connection with the embodiment is included in at least one embodiment of the disclosure. Furthermore, the particular features, structures or characteristics may be combined as suitable in one or more embodiments of the disclosure. The previous description is provided to enable any person skilled in the art to practice the various aspects described herein. Various modifications to these aspects will be readily apparent to those skilled in the art, and the generic principles defined herein may be applied to other aspects.

Although embodiments and features have been shown and described, it will be understood that they are not intended to limit the claimed invention, and it will be made obvious to those skilled in the art that various changes and modifications may be made without departing from the spirit and scope of the claimed invention. The specification and drawings are, accordingly to be regarded in an illustrative rather than restrictive sense. The claimed invention is intended to cover all alternatives, modifications, and equivalents.

**List of references**

| | | | |
|---|---|---|---|
| 10 | Dental object | 310 | Final 3D representation |
| 20 | Local device | 400 | Remote computing device |
| 30 | Scanning system | 450 | Processor(s) |
| 40 | Router | 460 | Computer readable medium |
| 42 | WAN connection | 500 | Start of scanning session |
| 44 | LAN connection | 502 | Establishing a connection |
| 46 | LAN connection | 504 | Obtaining scan data |
| 48 | LAN connection | 506 | Obtaining a performance indicator |
| 100 | Intraoral scanner | 508 | Evaluating the performance indicator |
| 102 | Distal end | 510 | Enter full scan mode |
| 104 | Proximal end | 512 | Transmitting the scan data |
| 106 | Scanner body | 514 | Receiving a digital representation |
| 108 | Scanner interface | 516 | Displaying the digital representation |
| 110 | Sleeve | 520 | Enter partial scan mode |
| 120 | Scanner battery | 522 | Transmitting reduced amount of scan data |
| 130 | Projector unit | 524 | Receiving a partial digital representation |
| 140 | Camera unit | 526 | Displaying the partial digital representation |
| 150 | Processor(s) | 530 | Ending scanning session |
| 160 | Computer readable medium | 540 | Identify a scan data timestamp |
| 170 | Scanner window | 542 | Identify a digital representation render timestamp |
| 180 | Mirror | 544 | Identify a digital representation displaying timestamp Subtract scan data timestamp from |
| 200 | Scanning station | 546 | the digital representation displaying timestamp |
| 202 | Display | 600 | Input event |
| 204 | Input device | 602 | Transmit input event |
| 250 | Processor(s) | 604 | Receive input event |
| 260 | Computer readable medium Digital representation or partial | 606 | Render frame based on input event |
| 300 | digital representation | 608 | Transmit result frame with TS1 in metadata |
| 302 | 3D visualization | 610 | Receive and display result frame (TS2) |
| 304 | Scan box | 612 | Determine event-to-display latency, TS2 - TS1 |
| 306 | Viewfinder image | | |

## Claims

1. A computer-implemented method for facilitating 3D intraoral scanning using cloud computing, the method comprising:
- obtaining scan data of a dental object at a local device, wherein the scan data comprises primary scan data indicative of the 3D geometry of the dental object of a patient, wherein the scan data comprises secondary scan data indicative of one or more characteristics other than the 3D geometry of the dental object;
- establishing a connection between the local device and a remote computing device;
- obtaining a performance indicator by detecting a performance of the connection;
- evaluating the performance indicator and based on the evaluation:
if the performance indicator exceeds a primary performance threshold, entering a full connection mode, wherein the full connection mode comprises:
• transmitting the scan data from the local device to the remote computing device,
• receiving, at the local device from the remote computing device, a digital representation of the dental object computed from the transmitted scan data, and
• displaying the digital representation,
if the performance indicator is less than the primary performance threshold entering a partial connection mode, wherein the partial connection mode comprises:
• transmitting a reduced amount of the scan data from the local device to the remote computing device,
• receiving, at the local device from the remote computing device, a partial digital representation of the dental object computed from the transmitted reduced amount of scan data, and
• displaying the partial digital representation.

2. The method of claim 1, wherein the partial connection mode comprises:
• generating the reduced amount of scan data by:
∘ compressing at least a part of the scan data, preferably the secondary scan data,
∘ reducing a resolution of the primary scan data, and/or
∘ omitting at least part of the secondary scan data from the scan data.

3. The method of claim 2, wherein compressing at least a part of the scan data is performed at a compression ratio determined based on the performance indicator.

4. The method of any of the previous claims, wherein the secondary scan data comprises color data indicative of one or more colors of the dental object.

5. The method of claims 2 and 4, wherein omitting at least part of the secondary scan data from the scan data comprises reducing the color depth of the color data or omitting the color data.

6. The method of claim 5, wherein the partial digital representation is in a reduced color depth or monochrome.

7. The method of claim 5, wherein one or more colors of the partial 3D representation is predicted at the remote computing device.

8. The method of any of the previous items and claim 4, wherein generating the reduced amount of scan data comprises:
∘ if the performance indicator is between the primary performance threshold and a secondary performance threshold which is lower than the primary performance threshold, compressing at least a part of the scan data, preferably all of the scan data, at a dynamic compression ratio selected based on the performance indicator, and
∘ if the performance indicator is less than the secondary performance threshold, omitting at least a part of the secondary scan data from the scan data.

9. The method of any of the previous claims, wherein detecting the performance of the connection comprises:
• transmitting time stamp data from the local device to the remote computing device, and receiving a result of transmission time measurement computed from the transmitted time stamp data from the remote computing device, and/or
• transmitting a return data package from the local device to the remote computing device, receiving, at the local device, the return data package from the remote computing device, and calculating a round-trip time based on the time difference between when the return data package was transmitted and received at the local device.

10. The method of any of the previous claims, wherein the partial connection mode comprises:
• processing at least a part of the secondary scan data into locally processed data at the local device,
• combining the received partial digital representation with the locally processed data, and
• displaying the partial digital representation with the locally processed data.

11. The method of any of the previous claims, wherein the partial digital representation has a lower resolution and/or color depth than the digital representation.

12. The method of any of the previous claims, wherein the partial connection mode comprises:
• processing at least a part of the secondary scan data into locally processed data at the local device,
• combining the received partial digital representation with the locally processed data, and
• displaying the partial digital representation with the locally processed data.

13. The method of any of the previous claims, wherein the method further comprises:
- displaying a first visual indication indicative of which of the full connection mode or the partial connection mode is active, and/or
- displaying a second visual indication indicative of switches between the full connection mode and the partial connection mode, and/or
- displaying a third visual indication indicative of the performance indicator.

14. A computer program product comprising instructions which, when the program is executed by a computer, causes the computer to carry out the method of any of the previous claims.

15. A scanning system comprising:
- an intraoral scanner configured for obtaining scan data of a dental object, wherein the scan data comprises primary scan data indicative of the 3D geometry of the dental object of a patient, wherein the scan data comprises secondary scan data indicative of one or more characteristics other than the 3D geometry of the dental object;
- a display;
- a connection module configured for establishing a connection between the intraoral scanner and the display and a remote computing device, and for obtaining a performance indicator by detecting a performance of the connection; and
- one or more processors configured for:
- evaluating the performance indicator and based on the evaluation:
if the performance indicator exceeds a primary performance threshold, entering a full connection mode, wherein the full connection mode comprises:
• transmitting the scan data to the remote computing device,
• receiving a digital representation of the dental object computed from the transmitted scan data from the remote computing device, and
• displaying the digital representation on the display,
if the performance indicator is less than the primary performance threshold entering a partial connection mode, wherein the partial connection mode comprises:
• transmitting a reduced amount of the scan data to the remote computing device,
• receiving a partial digital representation of the dental object computed from the transmitted reduced amount of scan data from the remote computing device, and
• displaying the partial digital representation on the display.
